(19) Europäisches Patentamt
European Patent Office
Office européen des brevets

(11) EP 0 780 157 B1

(12) EUROPÄISCHE PATENTSCHRIFT

(45) Veröffentlichungstag und Bekanntmachung des Hinweises auf die Patenterteilung:
**03.04.2002 Patentblatt 2002/14**

(51) Int Cl.[7]: **B01J 31/24**, B01J 31/18

(21) Anmeldenummer: **96120522.6**

(22) Anmeldetag: **19.12.1996**

(54) **Rutheniumkomplexe mit einem chiralen, zweizähnigen Phosphinoxazolin-Liganden zur enantioselektiven Transferhydrierung von prochiralen Ketonen**

Ruthenium complex with chiral bidentate phosphinoxazolin ligands for the enantioselective transfer hydrogenation of prochiral cetons

Complexe de ruthénium avec des ligands phosphinoxazoline chiral et bidentés pour l'hydrogénation transfert énantiosélective des cetones prochirales

(84) Benannte Vertragsstaaten:
**CH DE ES FR GB IT LI NL**

(30) Priorität: **22.12.1995 DE 19548399**

(43) Veröffentlichungstag der Anmeldung:
**25.06.1997 Patentblatt 1997/26**

(73) Patentinhaber: **BASF AKTIENGESELLSCHAFT
67056 Ludwigshafen (DE)**

(72) Erfinder:
- **Helmchen, Guenther,
69115 Heidelberg (DE)**
- **Langer, Thomas,
69221 Dossenheim (DE)**

(74) Vertreter: **Kinzebach, Werner, Dr. et al
Patentanwälte
Reitstötter, Kinzebach und Partner
Postfach 86 06 49
81633 München (DE)**

(56) Entgegenhaltungen:
**EP-A- 0 271 311**
**EP-A- 0 366 390**
**EP-A- 0 386 833**
**EP-A- 0 478 147**
**DE-A- 4 243 030**
**FR-A- 2 693 190**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft chirale Rutheniumkomplexe der allgemeinen Formel (I)

$$RuX^1X^2L^1L^2 \qquad (I)$$

mit einem chiralen Liganden $L^2$, die sich zur enantioselektiven Transferhydrierung prochiraler Ketone eignen.

Die enantioselektive Hydrierung von prochiralen Ketonen in Gegenwart chiraler Rhodium- oder Rutheniumkomplexe spielt eine große Rolle bei der Herstellung optisch aktiver Verbindungen. Die verwendeten Komplexe enthalten in den meisten Fällen einen chiralen Diphosphinliganden, dessen Herstellung umständlich ist und meist eine technisch aufwendige und teure Racematspaltung beinhaltet. Diese Nachteile machen eine industrielle Nutzung dieser Komplexe bislang schwierg und unwirtschaftlich. Die Verwendung von Wasserstoff als Reduktionsmittel wiederum erfordert in der Regel Hochdruckapparaturen, die hohe Betriebskosten verursachen.

Neben der katalytischen Hydrierung mit Wasserstoff ist die enantioselektive Transferhydrierungen in Gegenwart chiraler Rhodium- und Rutheniumkomplexe gebräuchlich. Die katalytisch aktiven Komplexe enthalten jedoch in der Regel ebenfalls aufwendig herzustellende Diphosphinliganden. Zudem kann bei Verwendung dieser Komplexe nur ein mäßiger Enantiomerenüberschuß erzielt werden. So beschreiben Genêt et al. (Synlett 1993, 478) einen Rutheniumkomplex mit chiralem Diphosphinliganden, der bestenfalls einen Enantiomerenüberschuß von 62% (ee) bei der Transferhydrierung von Acetophenon erbringt.

Noyori et al. (J. Am. Chem. Soc. 1995, 117, 7562) beschreiben einen Rutheniumkomplex mit dem chiralen N-(p-Tosyl)-1(s),2(s)-diphenylethylendiamin als Ligand, der als Katalysator hohe Enantiomerenüberschüsse bei einer Transferhydrierung prochiraler Ketone liefert. Die Raum-Zeit-Ausbeute ist allerdings eher mäßig und das System reagiert empfindlich auf sterische Hinderung im Substrat. So wird Acetophenon glatt, Isobutyrophenon hingegen nicht mehr reduziert.

Die DE 42 43 030 A beschreibt Komplexe von Übergangsmetallen, wie Ti, Zr, Fe, Ru, Rh, Ir, Ni, Pd, Pt, Cu und Zn mit einer heterocyclischen Imin-Elementverbindung als zweizähnigen, chiralen Liganden und einem zweiten organischen Liganden, wie $\eta^3$-Allyl, $\eta^5$-Cyclopentadienyl, CO, Ethylen, Norborndien, Cyclooctadien, Acetonitril und Benzonitril. Diese Übergangsmetallkomplexe werden zur enantioselektiven allylischen Substitution und auch als homogene Katalysatoren bei der katalytischen Hydrierung von Ketonen eingesetzt. Mathieu et al. (J. Chem. Soc. Chem. Comm. 1995, 1721) beschreiben einen Rutheniumkomplex mit einem zweizähnigen, chiralen Liganden, welcher P, N, O als Donoratome aufweist. Dieser Komplex stellt einen sehr effizienten Katalysator für die Transferhydrierung von Ketonen dar. Bei Verwendung prochiraler Ketone wird jedoch keine Enantioselektivität beobachtet.

Der Erfindung liegt daher die Aufgabe zugrunde, einen besseren Katalysator für die Transferhydrierung von prochiralen Ketonen zur Verfügung zu stellen. Insbesondere soll der Katalysator leicht zugänglich sein, hohe Enantioselektivität gewährleisten und für die Reduktion von Ketonen jeglicher Art brauchbar sein.

Diese Aufgabe wird überraschenderweise gelöst durch Komplexe der allgemeinen Formel (I),

$$RuX^1X^2L^1L^2 \qquad (I)$$

in der

$X^1$ und $X^2$ gleich oder verschieden sind und für ein Halogenidion, das Anion einer $C_1$-$C_{10}$-Carbonsäure, die gegebenenfalls 1, 2 oder 3 Chlor- oder Fluoratome trägt, das Anion der Methansulfinsäure oder der Trifluormethansulfinsäure stehen oder $X^1$ und $X^2$ zusammen für das Dianion einer 1,3-, 1,4- oder 1,5-Dicarbonsäure stehen,
$L^1$ für einen cyclischen Ether mit 5-6 Ringatomen,
einen Aromaten, der gegebenenfalls 1, 2 oder 3 Alkylgruppen als Substituenten aufweist,
$PF_3$, oder
einen Liganden der allgemeinen Formel (II)

$$PR^1R^2R^3 \qquad (II)$$

steht, worin $R^1$, $R^2$ und $R^3$ gleich oder verschieden sind und für eine Alkylgruppe, Cycloalkylgruppe oder Arylgruppe, die gegebenenfalls 1, 2 oder 3 Substituenten aufweist, die unabhängig voneinander ausgewählt sind unter $C_1$-$C_4$-Alkylgruppen oder $C_1$-$C_4$-Alkoxygruppen, stehen,

$L^2$ für einen zweizähnigen, chiralen Liganden der allgemeinen Formel (III) steht,

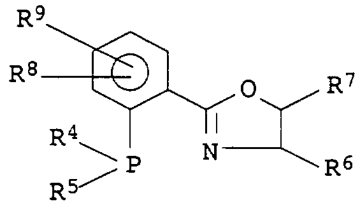

(III)

in der $R^4$ und $R^5$ gleich oder verschieden sind und für eine Alkylgruppe, Cycloalkylgruppe oder Arylgruppe, die gegebenenfalls 1, 2 oder 3 Substituenten aufweist, die unabhängig voneinander ausgewählt sind unter Alkylgruppen, Alkoxygruppen oder Halogenatomen, stehen, wobei einer der Reste $R^4$ oder $R^5$ auch ein Wasserstoffatom sein kann, oder in der

$R^4$ und $R^5$ zusammen mit dem Phosphoratom, an das sie gebunden sind, für einen gesättigten $C_5$-$C_7$-Ring, der gegebenenfalls mit einem oder zwei Arylkernen kondensiert ist, stehen,

$R^6$ für eine Alkylgruppe, die gegebenenfalls eine oder mehrere Gruppen aufweist, die ausgewählt sind unter OH, $NH_2$, SH, COOH, Aryloxy, Alkoxy, Arylthio, Alkylthio, Acyloxy, Alkoxycarbonyl, Acylamino,

oder eine Aralkylgruppe, Heteroaralkylgruppe, Arylgruppe oder Heteroarylgruppe steht,

$R^7$ für Wasserstoff, eine Alkyl-, Aryl- oder Aralkylgruppe steht,

$R^8$ und $R^9$ gleich oder verschieden sind und für Wasserstoff, $C_1$-$C_4$-Alkyl- oder für $C_1$-$C_4$-Alkoxygruppen stehen oder

$R^8$ und $R^9$ gemeinsam einen ankondensierten Cycloalkyl- oder Arylring bedeuten,

wobei die absolute Konfiguration an den Kohlenstoffatomen, die $R^6$ und $R^7$ tragen, unabhängig voneinander (S) oder (R) ist.

In der vorliegenden Anmeldung bedeutet Alkyl eine geradkettige oder verzweigte Alkylgruppe mit vorzugsweise 1 bis 6 und insbesondere 1 bis 4 Kohlenstoffatomen. Beispiele für Alkylgruppen sind Methyl, Ethyl, n-Propyl, i-Propyl, n-Butyl, i-Butyl, sek-Butyl und t-Butyl. Entsprechendes gilt für die Alkylgruppen in Alkoxy, Alkylthio, Alkoxycarbonyl etc. Cycloalkyl bedeutet vorzugsweise $C_5$-$C_8$-Cycloalkyl und insbesondere Cyclopentyl und Cyclohexyl. Aromat bzw. Aryl steht für Benzol, Naphthalin bzw. Phenyl, α- oder β-Naphthyl. $C_1$-$C_4$-Alkylsubstituierte Aromaten sind beispielsweise Toluol, Mesitylen oder p-Cymol. Bei den Heteroarylgruppen handelt es sich vorzugsweise 5- oder 6-gliedrige Heteroaromaten mit einem oder zwei Stickstoffatomen, die gegebenenfalls weitere Aromaten ankondensiert enthalten. Beispiele sind Pyrrolyl, Imidazolyl, Pyridyl, Pyrimidyl, Indolyl, Chinolinyl etc. Eine Aralkyl- bzw. Heteroaralkylgruppe steht für einen einwertigen Rest, in dem eine $C_1$-$C_4$-Alkylenkette eine Aryl- bzw. Heteroarylgruppe trägt. Acyl steht für RCO, wobei R insbesondere für H oder $C_1$-$C_4$-Alkyl steht.

Bei den $C_1$-$C_{10}$-Carbonsäuren, deren Anionen als Ligand $X^1$ oder $X^2$ im erfindungsgemäßen Komplex verwendet werden können, handelt es sich um lineare oder verzweigte Alkancarbonsäuren, die gegebenenfalls 1, 2 oder 3 Chlor- oder Fluoratome aufweisen oder um entsprechende Derivate der Benzoesäure oder Naphthoesäure. Bevorzugte Komplexe sind solche, in denen $X^1$ oder $X^2$ unabhängig voneinander für Halogenid, insbesondere Chlorid, Bromid und/oder Iodid oder das Anion einer $C_1$-$C_4$-Carbonsäure, insbesondere Acetat, Propionat und/oder Butyrat stehen. Ebenfalls bevorzugt werden die Anionen Trichloracetat, Trifluoracetat, Methansulfinat und Trifluormethansulfinat. Weiterhin können $X^1$ und $X^2$ zusammen für das Dianion einer 1,3-, 1,4- oder 1,5-Dicarbonsäure stehen. Hierunter sind Derivate der Malonsäure, der Bernsteinsäure oder der Glutarsäure zu verstehen.

Bevorzugte Liganden $L^1$ in den erfindungsgemäßen Komplexen mit der Formel (I) sind $PF_3$ und Phosphinliganden der allgemeinen Formel (II), in der die Gruppen $R^1$, $R^2$ und $R^3$ unabhängig voneinander vorzugsweise für lineare oder verzweigte $C_1$-$C_4$-Alkylgruppen oder Phenylgruppen, die gegebenenfalls durch 1, 2 oder 3 $C_1$-$C_4$-Alkylgruppen substituiert sind, stehen. Insbesondere handelt es sich um Methyl, Ethyl, n-Propyl, i-Propyl, n-Butyl und i-Butyl oder um Phenyl, ortho- oder para-Tolyl, para-Isopropylphenyl oder um Mesityl. Ganz besonders bevorzugte Komplexe enthalten als Liganden $L^1$ Triphenylphosphin, Tri-$C_1$-$C_4$-alkylphosphin, Tritolylphosphin oder Trimesitylphosphin.

Im Liganden $L^2$ mit der allgemeinen Formel (III) stehen die Gruppen $R^4$ und $R^5$ unabhängig voneinander vorzugsweise für $C_1$-$C_4$-Alkyl, insbesondere verzweigtes $C_1$-$C_4$-Alkyl, oder Aryl, das gegebenenfalls durch 1, 2 oder 3 $C_1$-$C_4$-Alkylgruppen substituiert ist. Besonders bevorzugt stehen $R^4$ und $R^5$ unabhängig voneinander für Methyl, Ethyl, n-Propyl, i-Propyl, n-Butyl, i-Butyl, Phenyl, ortho- oder para-Tolyl, Mesityl, α- oder β-Naphthyl. Bilden $R^4$ und $R^5$ gemeinsam mit dem P-Atom-, an das sie gebunden sind, einen heterocyclischen Ring, dann stehen $R^4$ und $R^5$ bevorzugt gemeinsam für n-Butylen, n-Pentylen oder 2,2'-Biphenylen.

Im Liganden $L^2$ mit der allgemeinen Formel (III) steht $R^6$ vorzugsweise für $C_1$-$C_4$-Alkyl, Phenyl, $(CH_2)_nX$, wobei X für Phenyl, 4-Imidazolyl, 3-Indolyl, α- oder β-Naphthyl, $NH_2$, OH, SH, $SCH_3$, COOH oder COO-$C_1$-$C_4$-Alkyl steht und n = 1, 2 oder 3 ist.

Insbesondere steht $R^6$ für Methyl, Ethyl, Propyl, i-Propyl, n-Butyl, 2-Butyl, i-Butyl, t-Butyl, Phenyl und Benzyl und besonders bevorzugt für i-Propyl und t-Butyl.

Erfindungsgemäß bevorzugte Atome oder Gruppen für $R^7$ sind Wasserstoff, Methyl oder Phenyl und besonders bevorzugt Wasserstoff.

Die absolute Konfiguration der Kohlenstoffatome im Oxazolinteil, die die Substituenten $R^6$ und $R^7$ tragen, kann unabhängig voneinander (R) oder (S) sein. Erfindungsgemäß kommen nur solche Liganden $L^2$ in Betracht, in denen die Konfiguration am C-Atom, das $R^6$ trägt, einheitlich (R) oder (S) ist.

Besonders bevorzugt ist der Ligand $L^2$ eine Verbindung der Formeln (IIIa), (IIIb) oder (IIIc) mit (R)- oder (S) -Konfiguration an dem C-Atom, das die Alkylgruppe trägt.

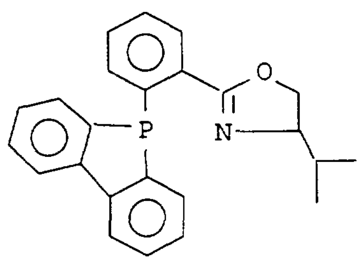

(IIIa)

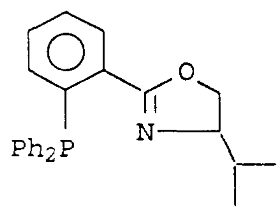

(IIIb)

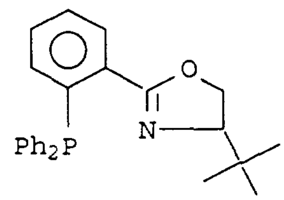

(IIIc)

Die Herstellung der erfindungsgemäßen Liganden $L^2$ ist bekannt oder kann analog zu bekannten Methoden erfolgen. Ihre Herstellung erfolgt beispielsweise ausgehend von chiralen Aminoalkoholen der allgemeinen Formel (V) durch eine sauer katalysiserte Ringschlußreaktion mit o-Fluorarylnitrilen der allgemeinen Formel (VI) zu den chiralen 2-(o-Fluoraryl)oxazolinderivaten der allgemeinen Formel (VII) (Bolm et al, Chem. Ber. 1991, 124, 1173). Diese lassen sich anschließend mit einem geeignet substituierten Phosphin-Anion zu dem gewünschten Liganden $L^2$ der allgemeinen Formel (III) umsetzen (Williamson et al, Synlett, 1993, 509). In den Verbindungen (III), (V), (VI) und (VII) besitzen die Gruppen $R^4$, $R^5$, $R^6$, $R^7$, $R^8$ und $R^9$ die oben genannte Bedeutung. Die chiralen Aminoalkohole sind beispielsweise ausgehend von chiralen Aminosäuren gemäß der in der oben genannten Veröffentlichung (Chem. Ber. 1991, 124, 1173) zitierten Literatur zugänglich.

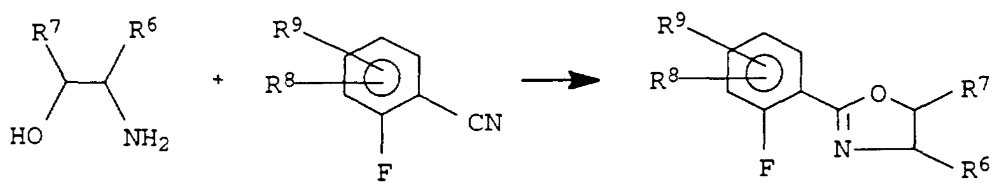

(V) (VI) (VII)

$$(VII) + M^{\oplus}R^4R^5P^{\ominus} \rightarrow (III)$$

Die Herstellung der erfindungsgemäßen Komplexe erfolgt durch Umsetzung eines chiralen Liganden der allgemeinen Formel (III) mit einem Vorläuferkomplex der Formel $RuX^1X^2L^1L^2L^3$, in der $X^1$, $X^2$ und $L^1$ die oben angezeigte Bedeutung besitzen, $L^2$ und $L^3$ gleich oder verschieden sind und ausgewählt werden unter Aromaten, die gegebenenfalls 1, 2 oder 3 Alkylsubstituenten aufweisen, insbesondere p-Cymol, oder Triarylphosphinen, insbesondere Triphenylphosphin. Die Liganden $L^2$ und $L^3$ können fehlen, insbesondere wenn die Vorläuferkomplexe in dimerer oder oligomerer Form vorliegen. Geeignete Komplexe sind beispielsweise $RuCl_2(PPh_3)_3$, $[RuCl_2(p\text{-}Cymol)]_2$. Die Umsetzung erfolgt in einem inerten Lösungsmittel, beispielsweise einem Aromaten, der gegebenenfalls 1, 2 oder 3 $C_1$-$C_4$-Alkylsubstituenten oder Chloratome trägt, vorzugsweise Benzol, Toluol, Ethylbenzol, p-Cymol, Chlorbenzol, Dichlorbenzol, oder einem Ether, vorzugsweise Diethylether, Tetrahydrofuran, Methyl-t-butylether, Anisol, oder einem Halogenalkan, beispielsweise Dichlormethan, Chloroform, Dichlorethan, Trichlorethan, bei Temperaturen im Bereich von 0°C bis 150°C, vorzugsweise von 10°C bis 100°C, besonders bevorzugt bei Raumtemperatur.

Die genannten Komplexe eignen sich als Katalysatoren für die Transferhydrierung von Ketonen der allgemeinen Formel (VIII).

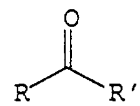

(VIII)

Sind R und R' verschieden, handelt es sich um prochirale Ketone und die durch die erfindungsgemäßen Komplexe katalysierte Transferhydrierung zu den entsprechenden Alkoholen ist enantioselektiv. Der Enantiomerenüberschuß beträgt mehr als 60% (ee), vorzugsweise mehr als 90% (ee).

Hinsichtlich der Art der Reste R und R' bestehen im Prinzip keine Beschränkungen. Sie stehen unabhängig voneinander für geradkettige oder verzweigte Alkyl-, Aryl-, (Hetero)aryl- oder (Hetero)aralkylgruppen, wobei alle Gruppen ihrerseits weitere Gruppen wie Alkyl-, (Hetero)aryl- oder (Hetero)aralkylgruppen aufweisen können. Vorzugsweise handelt es sich bei einem der Reste R und R' um eine Aryl- oder Heteroarylgruppe. Auch kann die zu reduzierende Carbonylfunktion in ein mono- oder polycyclisches Ringgerüst eingebaut sein. Weiterhin können die Reste R und R' unabhängig voneinander funktionelle Gruppen aufweisen. Für diese besteht lediglich die Einschränkung, daß sie nicht mit dem Katalysator unter Zerstörung desselben reagieren, oder daß sie nicht mit der zu reduzierenden Carbonylfunktion unter Bildung von Funktionalitäten abreagieren, die einer Transferhydrierung nicht mehr zugänglich sind. Diese Bedingungen erfüllen OH, SH, Aryloxy, Alkyloxy, Arylthio, Alkylthio, Acyloxy, COOH, Alkoxycarbonyl, Acylamino, CN, $NO_2$, Imine, olefinische Doppelbindungen und solche, die in Konjugation zu einem Elektronendonor (z.B. Enamine, Enolether) oder Elektronenakzeptor (Michael-Systeme) stehen.

Das erfindungsgemäße Verfahren zur enantioselektiven Transferhydrierung prochiraler Ketone erfolgt üblicherweise durch Umsetzung des Ketons mit einem Wasserstoff-Donor, vorzugsweise einem sekundären Alkohol, insbesondere i-Propanol, 2-Butanol, Cyclopentanol oder Cyclohexanol, speziell i-Propanol, in Gegenwart katalytischer Mengen eines oder mehrerer der erfindungsgemäßen Komplexe und katalytischer Mengen einer Base. Hierbei werden die Katalysatoren in Mengen von 0,001 bis 10 Mol% pro zu reduzierende Carbonylfunktion, vorzugsweise 0,01 bis 1 Mol%, insbesondere 0,05 bis 0,5 Mol% eingesetzt. Die Menge an Base, bezogen auf den Katalysator, liegt im Bereich von 0,5-100 Moläquivalenten, vorzugsweise 1-50 Mol-eq., insbesondere 5-30 Moleq. Geeignete Basen sind Alkalimetallhydroxide, Alkalimetall-$C_1$-$C_4$-alkoholate, Alkalimetallhydride oder Calciumhydrid. Vorzugsweise werden Alkalimetallhydroxide oder -alkoholate eingesetzt. Die Reaktion kann in dem sekundären Alkohol, sofern dieser flüssig ist, als Lösungsmittel durchgeführt werden. Weiterhin können auch, insbesondere wenn die Löslichkeit des Substrats dieses erfordert, Cosolventien eingesetzt werden, vorzugsweise Ether wie Diethylether, Tetrahydrofuran, Methyl-t-butylether, Dioxan, Pyran oder Kohlenwasserstoffe, Leichtbenzinfraktionen, vorzugsweise Hexan, sowie chlorierte Kohlenwasserstoffe, beispielsweise Dichlormethan oder Chloroform. Die Reaktion wird im Bereich von -30°C bis 150°C, vorzugsweise 20°C bis 100°C, und, falls das Lösungsmittel eine niedrigere Siedetemperatur besitzt, unter Druck durchgeführt. Die Reaktionsdauer liegt je nach Substrat und Temperatur im Bereich von 15 min. bis 12 h. Die Aufarbeitung der Reaktionsmischung erfolgt auf konventionellem Wege, die Gewinnung des Produkts erfolgt beispielsweise durch Destillation oder Kristallisation.

In einer alternativen Verfahrensvariante wird ein erfindungsgemäßer Rutheniumkomplex aus einem geeigneten Rutheniumvorläuferkomplex und einem Liganden $L^2$ der allgemeinen Formel (III) in einem inerten Lösungsmittel, z.B einem der erwähnten Cosolventien, generiert und anschließend das prochirale Keton, der sekundäre Alkohol und die Base zugefügt und die Mischung zur Reaktion gebracht.

Die im folgenden angegebenen Beispiele sollen die Erfindung erläutern, ohne sie einzuschränken.

Beispiele

Allgemeine Vorschrift zur Herstellung der erfindungsgemäßen Rutheniumkomplexe der allgemeinen Formel (I) mit $X^1 = X^2 = Cl$, $L^1 = P(C_6H_5)_3$

Zu einer Lösung von 0,3 mmol Tristriphenylphosphinruthenium(II)-chlorid $[RuCl_2(PPh_3)_3]$ in 6 ml wasserfreiem Toluol unter Inertgasatmosphäre gab man eine Lösung von 0,3 mmol eines Liganden $L^2$ der allgemeinen Formel (IIId) (siehe Tabelle 1) in 4 ml wasserfreiem Toluol und rührte ca. 12 h bei Raumtemperatur. Der ausgefallene Niederschlag

wurde abgesaugt, mit wenig Toluol gewaschen und im Vakuum getrocknet. Man erhielt auf diese Weise die in der nachfolgenden Tabelle 1 angegebenen Katalysatoren als braunes Pulver in einer Ausbeute von 55 bis 90%.

Alternativ entfernte man nach erfolgter Reaktion das Toluol vollständig im Vakuum, nahm den Rückstand in $CHCl_3$ auf und leitete die Kristallisation durch Überschichten mit Hexan ein.

Tabelle 1

| Katalysator $RuX^1X^2L^1L^2$ $X^1=X^2=Cl$ $L^1=PPh_3$ | Ligand $L^2$ 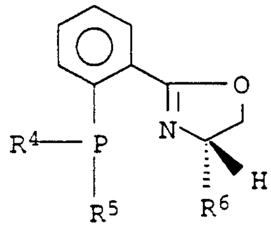 (IIId) | | | Ausbeute |
|---|---|---|---|---|
| | $R^4$ | $R^5$ | $R^6$ | |
| Bsp 1 | Phenyl | Phenyl | i-Propyl | 72% |
| Bsp 2 | 2,2'-Biphenylen | | i-Propyl | 79% |
| Bsp 3 | Phenyl | Phenyl | t-Butyl | 75% |
| Bsp 4 | Phenyl | Phenyl | Methyl | |
| Bsp 5 | α-Naphthyl | α-Naphthyl | i-Propyl | |

Die $^1H$- und $^{13}C$-NMR-Daten des Komplexes aus Beispiel 2 sind wie folgt:

$^1H$ NMR (300 MHz, $CDCl_3$) : δ = 0.69 [d,$J$=6.6Hz,3H,$CH(\underline{CH_3})_2$], 0.93 [d,$J$=6.7Hz,3H,$CH(\underline{CH_3})_2$], 2.05 [$m_c$,1H,$C\underline{H}(CH_3)_2$], 4.54 (t,$J$=8.6Hz,1H,$OCH_AH_B$), 4.85 (dd,$J$=8.6Hz,$J$=10.2Hz,1H,$OCH_AH_B$), 5.12 (dd,$J$=8.6Hz,$J$=8,3Hz, CHN=), 6.45 (dd,$J$=7.5Hz,$J$=8,2Hz,1H,Ar-H), 6.54 (d,$J$=3.6Hz,1H,Ar-H), 7.01-7.08 (m,7H,Ar-H), 7.19-7.36 (m, 13H,Ar-H), 7.51 (t,$J$=7.5Hz,1H,Ar-H), 7.62 (d,$J$=7.7Hz,1H,Ar-H), 7.78 (d,$J$=8.0Hz,1H,Ar-H), 7.87 (t,$J$=8.2Hz,1H, Ar-H), 8,18 (dd,$J$=3.9Hz,$J$=3.5Hz,1H,Ar-H).

$^{13}C$ NMR (75.5 MHz, $CDCl_3$): δ = 17.84 [t,$CH(CH_3)_2$], 23.06 [t,$CH(CH_3)_2$], 32.34 [d,$CH(CH_3)_2$], 70.74 (t,$OCH_2$), 75,07 (CHN=), 120.18, 120.28, 120.99 (s,C-Ar), 121.08, 127.26, 127.38, 127.84, 127.98 (d,C-Ar), 128.76, 128.89 (s,C-Ar), 129.56, 130.18, 130.65, 130.85 (d,C-Ar), 130.96 (s,C-Ar), 131.57, 131.67 (d,C-Ar), 132.20, 133.18, 133.33 (s,C-Ar), 133.74, (d,C-Ar), 134.95, 134.30 (s,C-Ar), 134.58, 134.71 (d,C-Ar), 140.27, 141.65 (s,C-Ar).

Allgemeine Vorschrift zur Reduktion von prochiralen Ketonen mit den erfindungsgemäßen Rutheniumkomplexen

Zu 5 ml 2-Propanol gab man nacheinander 0,01 mmol Katalysator (Tab. 2), 10 mmol Keton (Tab. 2) und 0,25 mmol festes KOH und erhitzte anschließend zum Rückfluß. Der Reaktionsverlauf wurde gaschromatographisch verfolgt. Anschließend destillierte man das gebildete Aceton und überschüssiges 2-Propanol ab. Zur Gewinnung des Wertprodukts unterwarf man den Rückstand einer Vakuumdestillation oder Kristallisation. Die erhaltenen Ergebnisse sind in Tabelle 2 zusammengestellt.

Tabelle 2

| Substrat | Katalysator | Reaktionsdauer | Wertprodukt | Ausbeute | (ee) |
|---|---|---|---|---|---|
| Acetophenon | Bsp 1 | 60 min | (R)-1-Phenylethanol | 83% | 94% |
| Isobutyrophenon | Bsp 2 | 30 min | (R)-1-Phenyl-2-methylpropanol | 83% | 93% |
| Cyclohexylmethylketon | Bsp 3 | 60 min | (S)-1-Cyclohexylethanol | 70% | 60% |

Alternativ kann die Transferhydrierung mit einem in situ-hergestellten Katalysator aus einem der Beispiele 1 bis 5 wie folgt durchgeführt werden.

0,01 mmol Tristriphenylphosphinruthenium(II)chlorid $[RuCl_2(PPh_3)_3$ werden in 0,5 ml wasserfreiem Toluol

vorgelegt, mit 0,013 mmol des Liganden $L^2$ (siehe Tabelle 1) vermischt und 0,5-20 h bei Raumtemperatur gerührt. Dann setzt man 5 ml Isopropanol, 10 mmol Keton und 0,25 mmol Base zu und erhitzt zum Rückfluß. Anschließend destilliert man das gebildete Aceton und überschüssiges 2-Propanol ab. Zur Gewinnung des Wertprodukts unterwirft man den Rückstand einer Vakuumdestillation oder Kristallisation.

**Patentansprüche**

1. Chirale Rutheniumkomplexe der allgemeinen Formel (I),

$$RuX^1X^2L^1L^2 \qquad (I)$$

in der

$X^1$ und $X^2$ gleich oder verschieden sind und für ein Halogenidion, das Anion einer $C_1$-$C_{10}$-Carbonsäure, die gegebenenfalls 1, 2 oder 3 Chlor- oder Fluoratome trägt, das Anion der Methansulfinsäure oder der Trifluormethansulfinsäure stehen oder
$X^1$ und $X^2$ zusammen für das Dianion einer 1,3-, 1,4- oder 1,5-Dicarbonsäure stehen,
$L^1$ für einen cyclischen Ether mit 5-6 Ringatomen,
einen Aromaten, der gegebenenfalls 1, 2 oder 3 Alkylgruppen als Substituenten aufweist,
$PF_3$, oder
einen Liganden der allgemeinen Formel (II)

$$PR^1R^2R^3 \qquad (II)$$

steht, worin $R^1$, $R^2$ und $R^3$ gleich oder verschieden sind und für eine Alkylgruppe, Cycloalkylgruppe oder Arylgruppe, die gegebenenfalls 1, 2 oder 3 Substituenten aufweist, die unabhängig voneinander ausgewählt sind unter $C_1$-$C_4$-Alkylgruppen oder $C_1$-$C_4$-Alkoxygruppen, stehen,
$L^2$ für einen zweizähnigen, chiralen Liganden der allgemeinen Formel (III) steht,

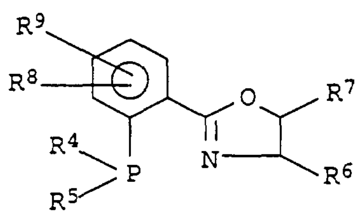

(III)

in der $R^4$ und $R^5$ gleich oder verschieden sind und für eine Alkylgruppe, Cycloalkylgruppe oder Arylgruppe, die gegebenenfalls 1, 2 oder 3 Substituenten aufweist, die unabhängig voneinander ausgewählt sind unter Alkylgruppen, Alkoxygruppen oder Halogenatomen, stehen, wobei einer der Reste $R^4$ oder $R^5$ auch ein Wasserstoffatom sein kann, oder in der
$R^4$ und $R^5$ zusammen mit dem Phosphoratom, an das sie gebunden sind, für einen gesättigten $C_5$-$C_7$-Ring, der gegebenenfalls mit einem oder zwei Arylkernen kondensiert ist, stehen,
$R^6$ für eine Alkylgruppe, die gegebenenfalls eine oder mehrere Gruppen aufweist, die ausgewählt sind unter OH, SH, $NH_2$, COOH Aryloxy, Alkoxy, Arylthio, Alkylthio, Acyloxy, Alkoxycarbonyl, Acylamino,
oder eine Aralkylgruppe, Heteroaralkylgruppe, Arylgruppe oder Heteroarylgruppe steht,
$R^7$ für Wasserstoff, eine Alkyl-, Aryl- oder Aralkylgruppe steht,
$R^8$ und $R^9$ gleich oder verschieden sind und für Wasserstoff, $C_1$-$C_4$-Alkyl- oder für $C_1$-$C_4$-Alkoxygruppen stehen oder
$R^8$ und $R^9$ gemeinsam einen ankondensierten Cycloalkyl- oder Arylring bedeuten,

wobei die absolute Konfiguration an den Kohlenstoffatomen, die $R^6$ und $R^7$ tragen, unabhängig voneinander (S) oder (R) ist.

**2.** Komplexe nach Anspruch 1 der allgemeinen Formel (I), in der $X^1$ und $X^2$ gleich oder verschieden sind und für Halogen oder das Anion einer $C_1$-$C_4$-Carbonsäure stehen.

**3.** Komplexe nach einem der vorhergehenden Ansprüche der allgemeinen Formel (I), worin $L^1$ ein Ligand der allgemeinen Formel (II) ist.

**4.** Komplexe nach Anspruch 3, wobei in Formel (II) $R^1$, $R^2$ und $R^3$ gleich oder verschieden sind und für $C_1$-$C_4$-Alkyl, oder Phenyl, das gegebenenfalls durch $C_1$-$C_4$-Alkyl substituiert ist, stehen.

**5.** Komplexe nach Anspruch 4, in denen $L^1$ für Triphenylphosphin, Tri-$C_1$-$C_4$-alkylphosphin, Tritolylphosphin oder Trimesitylphosphin steht.

**6.** Komplexe nach einem der vorhergehenden Ansprüche, wobei $R^4$ und $R^5$ in Formel (III) gleich oder verschieden sind und für $C_1$-$C_4$-Alkyl, insbesondere verzweigtes $C_1$-$C_4$-Alkyl, Aryl, das gegebenenfalls durch 1, 2 oder 3 $C_1$-$C_4$-Alkylgruppen substituiert ist, oder
$R^4$ und $R^5$ zusammen für n-Butylen, n-Pentylen oder 2,2'-Biphenylen stehen.

**7.** Komplexe nach einem der vorhergehenden Ansprüche, wobei $R^6$ in Formel (III) für $C_1$-$C_4$-Alkyl, Phenyl oder $(CH_2)_nX$ steht, wobei X für Phenyl, 4-Imidazolyl, 3-Indolyl, $\alpha$- oder $\beta$-Naphthyl, $NH_2$, OH, SH, $SCH_3$, COOH oder COO-$C_1$-$C_4$-Alkyl steht und n = 1, 2 oder 3 ist.

**8.** Komplexe nach einem der vorhergehenden Ansprüche der allgemeinen Formel (I), in der $X^1$ und $X^2$ für Halogen und $L^1$ für Triphenylphosphin stehen.

**9.** Komplexe nach einem der vorhergehenden Ansprüche, in dem der chirale, zweizähnige Ligand eine Verbindung der Formeln (IIIa), (IIIb) oder (IIIc) ist, wobei das C-Atom, das die Alkylgruppe trägt, einheitlich (R) oder (S) ist.

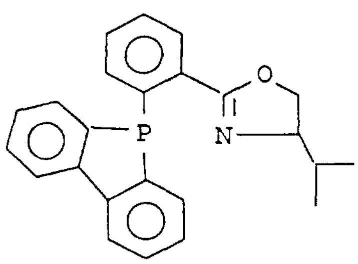

(IIIa)

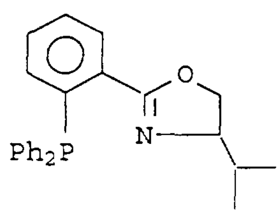

(IIIb)

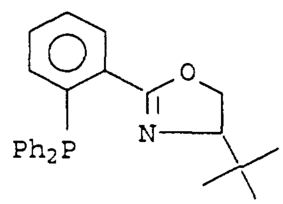

(IIIc)

**10.** Verfahren zur Herstellung der Komplexe nach einem der Ansprüche 1 bis 9, **dadurch gekennzeichnet, daß** man einen Vorläuferkomplex der allgemeinen Formel $RuX^1X^2L^1L^2L^3$, in der $X^1$, $X^2$ und $L^1$ die in Anspruch 1 angegebenen Bedeutungen besitzen und $L^2$, $L^3$ gleich oder verschieden sind und für einen Aromaten, Triarylphosphin, Trialkylphosphin, CO oder ein Dien stehen, in einem inerten Lösungsmittel mit dem jeweiligen Liganden der Formel (III) umsetzt.

**11.** Verwendung der Komplexe nach einem der Ansprüche 1 bis 9 zur enantioselektiven Transferhydrierung von prochiralen Ketonen.

**12.** Verwendung nach Anspruch 11, wobei man als Wasserstoffdonor einen sekundären Alkohol, insbesondere Isopropanol verwendet.

**13.** Verwendung nach Anspruch 11 oder 12, wobei man in Gegenwart katalytischer Mengen einer Base, insbesondere Alkalimetallhydroxiden oder -alkoholaten arbeitet.

## Claims

**1.** A chiral ruthenium complex of the formula (I),

$$RuX^1X^2L^1L^2 \qquad (I)$$

where

$X^1$ and $X^2$ are identical or different and are a halide ion, the anion of a $C_1$-$C_{10}$-carboxylic acid which may have 1, 2 or 3 chlorine or fluorine atoms, the anion of methanesulfinic acid or of trifluormethansulfinic acid, or $X^1$ and $X^2$ together are the dianion of a 1,3-, 1,4- or 1,5-dicarboxylic acid,
$L^1$ is a cyclic ether with 5-6 ring atoms,
an aromatic compound which may have 1, 2 or 3 alkyl groups as substituents,
$PF_3$, or
a ligand of the general formula (II)

$$PR^1R^2R^3 \qquad (II)$$

where $R^1$, $R^2$ and $R^3$ are identical or different and are an alkyl group, cycloalkyl group or aryl group, which may have 1, 2 or 3 substituents which are selected, independently of one another, from $C_1$-$C_4$-alkyl groups or $C_1$-$C_4$-alkoxy groups, $L^2$ is a bidentate, chiral ligand of the general formula (III),

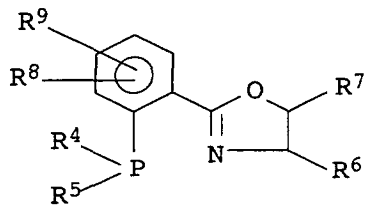
(III)

where $R^4$ and $R^5$ are identical or different and are an alkyl group, cycloalkyl group or aryl group, which may have 1, 2 or 3 substitutents which are selected, independently of one another, from alkyl groups, alkoxy groups or halogen atoms, it also being possible for one of the $R^4$ or $R^5$ radicals to be a hydrogen atom, or where $R^4$ and $R^5$ are, together with the phosphorus atom to which they are bonded, a saturated $C_5$-$C_7$-ring, which may be fused to one or two aryl nuclei,
$R^6$ is an alkyl group which may have one or more groups which are selected from OH, SH, $NH_2$, COOH, aryloxy, alkoxy, arylthio, alkylthio, acyloxy, alkoxycarbonyl, acylamino,
or an aralkyl group, hetaralkyl group, aryl group or hetaryl group,
$R^7$ is hydrogen, an alkyl, aryl or aralkyl group,
$R^8$ and $R^9$ are identical or different and are hyrogen, $C_1$-$C_4$-Alkyl or $C_1$-$C_4$-alkoxy groups, or
$R^8$ and $R^9$ together are a fused cycloalkyl or aryl ring,

where the absolute configuration at the carbon atoms which carry $R^6$ and $R^7$ is (S) or (R) independently of one another.

**2.** A complex as claimed in claim 1 of the formula (I) were $X^1$ and $X^2$ are identical or different and are halogen or the anion of a $C_1$-$C_4$-carboxylic acid.

**3.** A complex as claimed in either of the preceding claims of the formula (I), where $L^1$ is a ligand of the formula (II).

**4.** A complex as claimed in claim 3, wherein in formula (II) $R^1$, $R^2$ and $R^3$ are identical or different and are $C_1$-$C_4$-alkyl, or phenyl, which is unsubstituted or substituted by $C_1$-$C_4$-alkyl.

**5.** A complex as claimed in claim 4, where $L^1$ is triphenylphosphine, tri-$C_1$-$C_4$-alkylphosphine, tritolylphosphine or trimesitylphosphine.

**6.** A complex as claimed in any of the preceding claims, where $R^4$ and $R^5$ in formula (III) are identical or different and are $C_1$-$C_4$-alkyl, in particular branched $C_1$-$C_4$-alkyl, aryl, which is unsubstituted or substituted by 1, 2 or 3 $C_1$-$C_4$-alkyl groups, or
$R^4$ and $R^5$ together are n-butylene, n-pentylene or 2,2'-biphenylene.

**7.** A complex as claimed in any of the preceding claims, wherein $R^6$ in the formula (III) is $C_1$-$C_4$-alkyl, phenyl or $(CH_2)_nX$, where X is phenyl, 4-imidazolyl, 3-indolyl, $\alpha$- or $\beta$-naphthyl, $NH_2$, OH, SH, $SCH_3$, COOH or COO-$C_1$-$C_4$-alkyl, and n is 1, 2 or 3.

**8.** A complex as claimed in any of the preceding claims of the formula (I) where $X^1$ and $X^2$ are halogen and $L^1$ is triphenylphosphine.

**9.** A complex as claimed in any of the preceding claims, in which the chiral, bidentate ligand is a compound of the formulae (IIIa), (IIIb) or (IIIc), where the carbon atom which carries the alkyl group is uniformly (R) or (S).

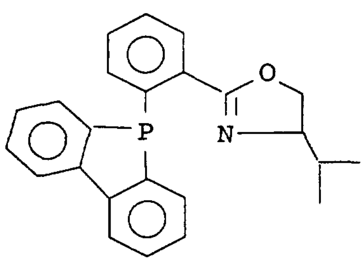

(IIIa)

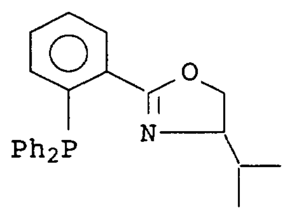

(IIIb)

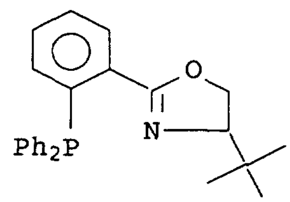

(IIIc)

**10.** A process for preparing the complexes as claimed in any of claims 1 to 9, which comprises reacting a precursor complex of the general formula $RuX^1X^2L^1L^2L^3$, where $X^1$, $X^2$ and $L^1$ have the meanings indicated in Claim 1, and $L^2$, $L^3$ are identical or different and are an aromatic compound, triarylphosphine, trialkylphosphine, CO or a diene, in an inert solvent with the particular ligand of the formula (III).

**11.** The use of the complexes as claimed in any of claims 1 to 9 for the enantioselective transfer hydrogenation of prochiral ketones.

**12.** The use as claimed in claim 11, wherein a secondary alcohol, in particular isopropanol, is used as hydrogen donor.

**13.** The use as claimed in claim 11 or 12, wherein catalytic amounts of a base, in particular alkali metal hydroxides or alcoholates, are present.

**Revendications**

**1.** Complexes chiraux de ruthénium de formule générale (I) ,

$$RuX^1X^2L^1L^2 \qquad (I)$$

où

$X^1$ et $X^2$ sont identiques ou différents et désignent l'ion halogénure, l'anion d'un acide carboxylique en $C_1$-$C_{10}$, qui porte éventuellement 1, 2 ou 3 atomes de chlore ou de fluor, l'anion de l'acide méthane-sulfinique ou de

l'acide trifluorométhanesulfinique, ou
$X^1$ et $X^2$ représentent ensemble le dianion d'un acide 1,3-, 1,4- ou 1,5-dicarboxylique,
$L^1$ représente un éther cyclique ayant 5-6 atomes de cycle, un groupe aromatique qui présente éventuellement 1, 2 ou 3 groupes alkyle à titre de substituants,
PF3, ou
un ligand de formule générale (II)

$$PR^1R^2R^3 \qquad (II)$$

où $R^1$, $R^2$ et $R^3$ sont identiques ou différents et désignent un groupe alkyle, un groupe cycloalkyle ou un groupe aryle, qui présente éventuellement 1, 2 ou 3 substituants, qui sont choisis indépendamment l'un de l'autre parmi les groupes alkyle en $C_1$-$C_4$ ou les groupes alcoxy en $C_1$-$C_4$,
$L^2$ représente un ligand chiral, bidenté de formule générale (III),

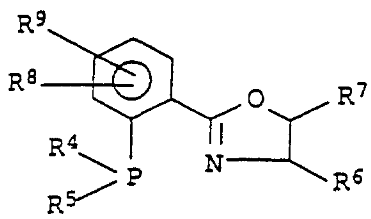

(III)

dans laquelle $R^4$ et $R^5$ sont identiques ou différents et désignent un groupe alkyle, un groupe cycloalkyle ou un groupe aryle, qui présente éventuellement 1, 2 ou 3 substituants, qui sont choisis indépendamment l'un de l'autre parmi les groupes alkyle, les groupes alcoxy ou les atomes d'halogène, tandis que l'un des restes $R^4$ ou $R^5$ peut également être un atome d'hydrogène, ou dans laquelle $R^4$ et $R^5$ désignent, conjointement avec l'atome de phosphore auquel ils sont liés, un cycle saturé en $C_5$-$C_7$, qui est éventuellement condensé avec un ou deux noyaux aryle, $R^6$ représente un groupe alkyle, qui présente éventuellement un ou plusieurs groupes, qui sont choisis parmi OH, SH, $NH_2$, COOH, aryloxy, alcoxy, arylthio, alkylthio, acyloxy, alcoxycarbonyle, acylamino,
ou un groupe aralkyle, un groupe hétéroaralkyle, un groupe aryle ou un groupe hétéroaryle,
$R^7$ désigne l'hydrogène, ou un groupe alkyle, aryle ou aralkyle,
$R^8$ et $R^9$ sont identiques ou différents et représentent l'hydrogène ou des groupes alkyle en $C_1$-$C_4$ ou alcoxy en $C_1$-$C_4$, ou
$R^8$ et $R^9$ pris ensemble représentent un cycle cycloalkyle ou aryle condensé,

tandis que la configuration absolue sur les atomes de carbone que portent $R^6$ et $R^7$ est, indépendamment l'une de l'autre, (S) ou (R).

2. Complexes selon la revendication 1 de formule générale (I), où $X^1$ et $X^2$ sont identiques ou différents et désignent un halogène ou l'anion d'un acide carboxylique en $C_1$-$C_4$.

3. Complexes selon l'une des revendications précédentes de formule générale (I), où $L^1$ est un ligand de formule générale (II).

4. Complexes selon la revendication 3, tandis que dans la formule (II) $R^1$, $R^2$ et $R^3$ sont identiques ou différents et désignent un alkyle en $C_1$-$C_4$, ou un phényle, qui est éventuellement substitué par un alkyle en $C_1$-$C_4$.

5. Complexes selon la revendication 4, dans lesquels $L^1$ désigne la triphénylphosphine, la trialkyl($C_1$-$C_4$)-phosphine, la tritolylphosphine ou la trimésitylphosphine.

6. Complexes selon l'une des revendications précédentes, où $R^4$ et $R^5$ dans la formule (III) sont identiques ou différents et désignent un alkyle en $C_1$-$C_4$, en particulier un alkyle en $C_1$-$C_4$ ramifié, un aryle, qui est éventuellement substitué par 1, 2 ou 3 groupes alkyle en $C_1$-$C_4$, ou

$R^4$ et $R^5$ désignent ensemble un n-butylène, un n-pentylène ou un 2,2'-biphénylène.

**7.** Complexes selon l'une des revendications précédentes, où $R^6$ dans la formule (III) désigne un alkyle en $C_1$-$C_4$, un phényle ou $(CH_2)_nX$, tandis que X représente un groupe phényle, 4-imidazolyle, 3-indolyle, α- ou β-naphtyle, $NH_2$, OH, SH, $SCH_3$, COOH ou COO-alkyle en $C_1$-$C_4$ et n = 1, 2 ou 3.

**8.** Complexes selon l'une des revendications précédentes de formule générale (I), dans laquelle $X^1$ et $X^2$ désignent un groupe halogéno et $L^1$ la triphénylphosphine.

**9.** Complexes selon l'une des revendications précédentes, dans lesquels le ligand bidenté, chiral est un composé de formules (IIIa), (IIIb) ou (IIIc), tandis que l'atome de carbone qui porte le groupe alkyle est uniformément (R) ou (S).

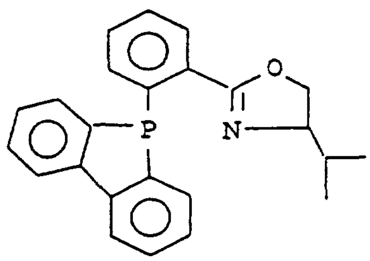

(IIIa)

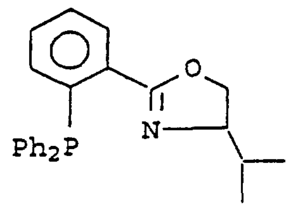

(IIIb)

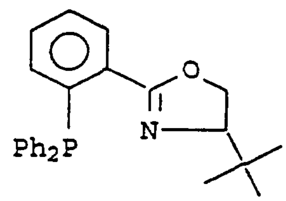

(IIIc)

**10.** Procédé pour la préparation de complexes selon l'une des revendications 1 à 9, **caractérisé par le fait qu'**on fait réagir un complexe précurseur de formule générale $RuX^1X^2L^1L^2L^3$, dans laquelle $X^1$, $X^2$ et $L^1$ possèdent les significations indiquées dans la revendication 1 et $L^2$, $L^3$ sont identiques ou différents et représentent un groupe aromatique, triarylphosphine, trialkylphosphine, CO ou un diène, dans un solvant inerte avec le ligand correspondant de formule (III).

**11.** Utilisation des complexes selon l'une des revendications 1 à 9 pour l'hydrogénation transfert énantiosélective de cétones prochirales.

**12.** Utilisation selon la revendication 11, tandis qu'on utilise comme donneur d'hydrogène un alcool secondaire, en particulier l'isopropanol.

**13.** Utilisation selon la revendication 11 ou 12, tandis qu'on opère en présence de quantités catalytiques d'une base, en particulier d'hydroxyde ou d'alcoolates de métal alcalin.